# EUROPÄISCHE PATENTSCHRIFT

(11) **EP 1 198 460 B1**
(45) Veröffentlichungstag und Bekanntmachung des Hinweises auf die Patenterteilung: **23.03.2005**
(21) Anmeldenummer: 00954561.7
(22) Anmeldetag: 25.07.2000
(51) Int. Cl.: C07D 251/62, C07D 251/60

(54) **VERFAHREN ZUR HERSTELLUNG VON FESTEM MELAMIN**
METHOD FOR PRODUCING SOLID MELAMINE
PROCEDE DE FABRICATION DE MELAMINE SOLIDE

(30) Priorität: 27.07.1999 AT 129899
(43) Veröffentlichungstag der Anmeldung: 24.04.2002
(73) Patentinhaber: AMI Agrolinz Melamine International GmbH, 4020 Linz (AT)
(72) Erfinder: COUFAL, Gerhard, A-4060 Leonding (AT)
(74) Vertreter: Gross, Felix
(86) Internationale Anmeldenummer: PCT/EP2000/007092
(87) Internationale Veröffentlichungsnummer: WO 2001/007420

(56) Entgegenhaltungen:
- WO-A-96/20183
- WO-A-97/20826
- WO-A-98/54160
- WO-A-98/55466

## Beschreibung

Die Erfindung betrifft ein Verfahren zur Herstellung von festem Melamin, bei dem eine NH₃-hältige Melaminschmelze verfestigt, anschließend entspannt und rasch abgekühlt wird.

Melamin wird bevorzugt durch Pyrolyse von Harnstoff hergestellt, wobei sowohl Niederdruckverfahren als auch Hochdruckverfahren, wie sie beispielsweise in "Ullmann's Encyclopedia of Industrial Chemistry, Vol A 16, 5^{th} ed (1990), Seiten 171-185" beschrieben sind, zur Anwendung kommen können. Das bei der Melaminsynthese anfallende Melamin enthält je nach Herstellverfahren etwa 94-98 Gew.% Melamin, sowie insbesondere Melam, Melem, Ureidomelamin, Ammelin und Ammelide als wesentliche Nebenprodukte bzw. Verunreinigungen und muß für anspruchsvollere Anwendungsgebiete durch besondere Verfahrensschritte weiter gereinigt werden. Dabei ist es wesentlich, daß zur Verhinderung der Rückbildung von Nebenprodukten, bei höheren Temperaturen immer ein hoher NH₃-Druck aufrecht erhalten wird. Es darf demnach erst nach entsprechender Abkühlung entspannt werden (JP-A-46-16987).
Um Melamin in fester Form zu erhalten, kann die flüssige Melaminschmelze beispielsweise gemäß US 4,565,867 mit Ammoniak, oder gemäß WO 99/38852 in einem Wirbelbett mit kalten festen Inertstoffen oder festem Melamin abgekühlt werden. Es ist auch möglich, eine ammoniakhältige Melaminschmelze beispielsweise gemäß W097/20826 in einen Abkühlbehälter mit Ammoniakatmosphäre einzusprühen und zu entspannen, wobei sich festes Melamin in reiner Form abscheidet. Wie beispielsweise in W098/54160 beschrieben, wird das feste Melamin dann unter Druck weiter gekühlt und erst im Anschluß an die Kühlung weiter entspannt. Dabei besteht vor allem der Nachteil, daß die Abkühlung bei hohem Druck in speziellen Druckapparaten erfolgen muß.
Es konnte nun unerwarteterweise gefunden werden, daß es auch dann möglich ist, Melamin in reiner Form mit maximal etwa 0,1 bis 0,2 Gew. % an Verunreinigungen zu erhalten, wenn das feste Melamin entspannt wird und gleichzeitig oder unmittelbar anschließend an die Entspannung rasch gekühlt wird.

Gegenstand der Erfindung ist demnach ein Verfahren zur Herstellung von festem Melamin, dadurch gekennzeichnet, daß
a) eine NH₃-hältige Melaminschmelze durch Kühlen und/oder Entspannen, mit oder ohne NH₃-Zufuhr verfestigt wird,
b) das erhaltene feste Melamin bei Temperaturen zwischen etwa 300 °C und seinem vom jeweiligen NH₃-Druck abhängigen Schmelzpunkt bei Drücken von etwa 10 bis 300 bar während 1 min bis 5 h verweilen gelassen wird,
c) anschließend auf einen Druck von etwa 1 bis 15 bar, bevorzugt auf etwa 1 bis 10 bar, entspannt wird,
d) rasch innerhalb von 0 bis 10 min auf eine Temperatur von unter 280 °C gekühlt wird und
e) anschließend in beliebiger Reihenfolge weiter gekühlt und weiter entspannt wird.

Das erfindungsgemäße Verfahren kann sowohl diskontinuierlich als auch kontinuierlich durchgeführt werden. Die NH₃-hältige Melaminschmelze wird beispielsweise bei etwa 50 - 800 bar und etwa 325 bis 450 °C in einem Melamin-Hochdruckreaktor aus Harnstoff gebildet, anschließend werden die im wesentlichen aus NH₃, CO₂ und Melamindampf bestehenden Offgase abgetrennt, in einem Harnstoffwäscher vom Melamin befreit und in eine Harnstoffanlage rückgeführt. Nach Abtrennen der Offgase kann die Melaminschmelze beispielsweise mit NH₃ gestrippt werden, wodurch vor allem restliches CO₂ entfernt wird. Zur Erzielung einer besonders guten Melaminqualität ist es vorteilhaft, die flüssige Melaminschmelze unter Ammoniakdruck in einem Aging-Behälter oder einem Röhrenreaktor verweilen zu lassen. Der Druck beim Aging liegt im Bereich von etwa 50 bis 1000 bar, bevorzugt bei etwa 80 bis 600 bar, besonders bevorzugt bei etwa 130 - 400 bar.

Anschließend kann gegebenenfalls mit oder ohne NH₃-Zufuhr auf eine Temperatur, die etwa 1 bis 50 °C, bevorzugt etwa 1 bis 20 °C über dem vom jeweiligen Ammoniakdruck abhängigen Schmelzpunkt des Melamins liegt, gekühlt werden. Dabei ist es vorteilhaft, die Temperatur des flüssigen Melamins beispielsweise durch Zufuhr von flüssigem, gasförmigem oder überkritischem NH₃ abzusenken. Grundsätzlich kann die Temperatur des zu kühlenden flüssigen Melamins in einem großen Bereich variieren. Sie liegt oberhalb des vom jeweiligen Ammoniak-Druck abhängigen Schmelzpunktes von Melamin, üblicherweise jedoch unterhalb von etwa 400°C, bevorzugt unterhalb von etwa 370 °C, besonders bevorzugt unterhalb von etwa 350 °C. Je höher der Ammoniakdruck ist und je niedriger die Temperatur der Melaminschmelze ist, umso mehr Ammoniak ist im Melamin enthalten, und umso niedriger ist der Schmelzpunkt. Es ist also auch möglich, bei 300 °C und darunter flüssiges Melamin, genauer gesagt, eine Mischung von flüssigem Melamin mit Ammoniak vorliegen zu haben und zu entspannen, falls der Druck hoch genug ist. Besonders vorteilhaft ist es, bei einer Temperatur, die nicht wesentlich über dem jeweiligen Schmelzpunkt des Melamins liegt, in den Wirbelschichtapparat zu entspannen. Die Abkühlung bis knapp oberhalb des Schmelzpunktes des Melamins erfolgt bevorzugt durch Zufuhr von kaltem flüssigem oder gasförmigem, bzw. von überkritischem Ammoniak. Das im flüssigen Melamin enthaltene Ammoniak trägt beim nachfolgenden Entspannen ebenfalls zur Abkühlung bei und wirkt der beim Erstarren des Melamins freiwerdenden Schmelzenthalpie entgegen.
Der NH₃-Druck über der zu kühlenden Melaminschmelze kann in einem großen Bereich variieren. Häufig liegt er beim Druck der im Reaktor durchgeführten Melaminsynthese. Er kann jedoch wesentlich höher liegen, wenn der Melaminsynthese ein "Aging" bei höherem Druck nachgeschaltet ist. Der Druck kann demnach bis zu 1000 bar oder bis zu den ökonomisch und materialmäßig sinnvollen und möglichen Grenzen betragen.

Anschließend kann die NH₃-hältige Melaminschmelze, die bevorzugt mit NH₃ gesättigt ist, beispielsweise durch Quenchen mit flüssigem, gasförmigem oder superkritischem NH₃, gegebenenfalls unter gleichzeitiger Entspannung, oder durch Entspannung mit oder ohne NH₃-Zufuhr verfestigt werden.

Besonders vorteilhaft erfolgt die Verfestigung gemäß PCT/EP99/00353 in einem mit NH₃ als Kreislaufgas betriebenen Wirbelbett, in das die NH₃-hältige Melaminschmelze eingetragen und auf den dort herrschenden Druck entspannt, mit festem Melamin und/oder mit festen Inertstoffen, beispielsweise Keramik-, Glas- oder Metallpartikeln gekühlt und verfestigt wird. Dabei wird die Schmelze über Düsen auf das wirbelnde Gut derart verteilt, daß die Oberfläche der kalten Feststoffteilchen benetzt wird.
Ein weiterer Gegenstand der Erfindung ist demnach ein Verfahren zur Herstellung von festem Melamin, bei dem
a) eine NH₃-hältige Melaminschmelze in einem mit im wesentlichen aus NH₃ als Kreislaufgas betriebenen Wirbelbett durch Entspannung und Kühlung mit festem Melamin und/oder festen Inertstoffen verfestigt wird,
b) das erhaltene feste Melamin bei Temperaturen zwischen etwa 300 °C und seinem vom jeweiligen NH₃-Druck abhängigen Schmelzpunkt bei Drücken von etwa 10 bis 300 bar während 1 min bis 5 h verweilen gelassen wird,
c) anschließend auf einen Druck von etwa 1 bis 15 bar, bevorzugt auf etwa 1 bis 10 bar, entspannt wird,
d) rasch innerhalb von 0 bis 10 min auf eine Temperatur von unter 280 °C gekühlt wird und
e) anschließend in beliebiger Reihenfolge weiter gekühlt und weiter entspannt wird.

Im Falle der Verwendung von festem Melamin als Kühlmedium bzw. als Kristallisationskeime im Wirbelbett erstarrt die Schmelze an deren Oberfläche, wodurch die Melaminteilchen anwachsen und sobald sie eine bestimmte Größe erreicht haben, aufgrund ihres Gewichtes durch eine klassierende Austragsvorrichtung aus der Wirbelschicht abgezogen werden. Durch die Kühlung im Wirbelbett wird bei sehr gutem Wärme- und Stoffübergang ein sehr einheitliches, annähernd kugelförmiges und weitgehend staubfreies Melamingranulat mit guter Rieselfähigkeit erhalten. Um die Zahl der Feststoffpartikel im Wirbelbett konstant zu halten, werden je nach Ausgestaltung des Wirbelbettes und je nach Verfahrensführung laufend neue Feststoffteilchen zugegeben, die als Kristallisationskeime dienen und zu neuem Granulat anwachsen. Es bilden sich aber auch durch einen gewissen Abrieb im Wirbelbett laufend neue Granulat- bzw. Kristallisationskeime, von denen die Granulatbildung ausgehen kann. Die Temperatur des festen Melamins in der Wirbelschicht kann bei jedem beliebigen Wert unterhalb des Schmelzpunktes von Melamin liegen, wobei eine größere Temperaturdifferenz zwischen festem und zu kühlendem, flüssigem Melamin einen größeren Kühleffekt hat.

Im Falle der Kühlung mittels fester Inertstoffe erstarrt das flüssige Melamin an der Oberfläche der Inertstoffe. Einerseits wächst die Schicht an festem Melamin über den Inertstoffen, andererseits wird das aufgewachsene feste Melamin dieser beschichteten Inertstoffpartikel durch das Reiben aneinander ständig abgerieben. Das abgeriebene feste Melamin wird mit dem Wirbelgas ausgetragen und beispielsweise über einen Zyklon abgeschieden.

Die im Wirbelbett vorhandene und aufrechterhaltene Temperatur kann je nach gewählter Verfahrensweise in einem großen Bereich zwischen Raumtemperatur und bis knapp unterhalb des druckabhängigen Schmelzpunktes von Melamin schwanken. Sie beträgt beispielsweise etwa 100 bis etwa 340 °C, bevorzugt etwa 200 bis etwa 340 °C, besonders bevorzugt etwa 280 bis etwa 320 °C. Die Temperatursteuerung im Wirbelbett kann auf mehrfache Weise erfolgen, beispielsweise durch eingebaute Kühlelemente, durch Zufuhr von festem kaltem Melamin, durch gegebenenfalls ausgeschleuste und nach externer Kühlung wieder in das Wirbelbett rückgeführte Inertpartikel, durch Zufuhr von kaltem flüssigem oder gasförmigem NH₃, durch die Temperatur und Menge des Gasstromes, mit dem die Wirbelschicht aufrechterhalten wird und durch die Verdampfungsenthalpie des im flüssigen Melamin enthaltenen Ammoniaks. Ein Teil des Ammoniaks wird, zur Kühlung und zur Aufrechterhaltung des Wirbelbettes, im Kreislauf geführt. Der andere Teil des freiwerdenden Ammoniaks kann je nach vorhandenem Druck im Wirbelbett gasförmig oder verflüssigt in den Melamin/Harnstoffprozeß rückgeführt werden.
Der im Wirbelschichtreaktor vorhandene Druck kann je nach gewählter Verfahrensweise ebenfalls in einem großen Bereich schwanken. Er kann von zwischen etwas über 1 bar bis knapp unterhalb des Druckes der zu kühlenden Melaminschmelze betragen. Üblicherweise beträgt der Druck im Wirbelschichtreaktor zwischen etwa 1,5 und etwa 100 bar, bevorzugt zwischen etwa 1,5 bar und 50 bar, besonders bevorzugt zwischen etwa 5 bis 25 bar. Bei einem Druck von über etwa 13 bar kann das überschüssige NH₃-Gas leicht verflüssigt und in die Harnstoff- und Melaminsynthese rückgeführt werden.

Die Temperatur des aus dem Wirbelbett ausgetragenen festen Melamins kann jeden Wert unterhalb des Schmelzpunktes von Melamin betragen.

Das gemäß a) erhaltene feste Melamin wird bei Temperaturen von etwa 300 °C bis zu seinem vom jeweiligem NH₃-Druck abhängigen Schmelzpunkt und bei Drücken von etwa 10 bis 300 bar während 1 min bis 5 h im Festzustand verweilen gelassen (getempert). Bevorzugt wird dabei so nahe wie möglich, etwa 1 bis 5 °C unterhalb des vom jeweils herrschenden NH₃-Druck abhängigen Schmelzpunktes des Melamins getempert.

Für den Fall, daß kein Tempern erfolgt, wird das feste Melamin erfindungsgemäß ebenfalls von diesen für das Tempern geltenden Druck- und Temperaturbedingungen auf etwa 1 bis 15 bar entspannt und rasch auf unter 280 °C gekühlt. Bevorzugt wird auf etwa 1 bis 10 bar, besonders bevorzugt auf etwa 1 bis 5 bar, bzw. auf Atmosphärendruck (1 bar) entspannt. Die Temperatur nach dem Entspannen liegt bevorzugt unter 250 °C, besonders bevorzugt unter 200 °C bzw. unter 100 °C. Erfindungsgemäß ist es wesentlich, daß die Kühlung unmittelbar im Anschluß an die Entspannung erfolgt, bevorzugt innerhalb von 10 sec bis 5 min, besonders bevorzugt innerhalb von 1 min bis 2 min. Wesentlich dabei ist, daß die Kühlung umso rascher erfolgen muß, je höher die Temperatur nach der Entspannung und je niedriger der Druck nach der Entspannung sind. Um eine gute Melaminqualität, insbesondere niedere APHA-Werte als Maß für die Gelbstichigkeit des Melamins und einen niedrigen Gehalt vor allem an Melam zu erreichen, ist es beispielsweise beim Entspannen bis auf Atmosphärendruck notwendig, innerhalb von höchstens etwa 2 min auf unter 280 °C zu kühlen.

Das gemäß c) erhaltene feste Melamin kann gemäß d) vorteilhaft in einem mit im wesentlichen aus NH₃ als Kreislaufgas betriebenen Wirbelbett mit kaltem, festem Melamin und/oder kalten festen Inertstoffen gekühlt werden. Dabei können auch die besonderen Vorteile des Wirbelbettes, nämlich die guten Wärme- und Stoffübergänge, für eine rasche Kühlung optimal ausgenützt werden. Die Kühlung im Wirbelbett und damit die Einstellung der gewünschten Temperatur im Wirbelbett kann in analoger Weise zur Kühlung des Wirbelbettes gemäß a) zur Verfestigung der Melaminschmelze erfolgen.
Das gemäß c) erhaltene feste Melamin kann gemäß d) auch in Kühlapparaten mit flüssigem NH₃ oder mit kalten Gasen, wie z.B. NH₃, Stickstoff oder Luft gekühlt werden. Weiters ist es möglich, das gemäß c) erhaltene feste Melamin gemäß d) mittels spezieller Kühlelemente, Wärmetauscher, Kühlflächen, Kühlmischer oder Kühlschnecken, z.B. Pflugscharmischer oder Mischer von beispielsweise List, Lödige, Drais oder Buss, zu kühlen. Dabei ist es auch vorteilhaft, zusätzlich kalte Gase oder flüssiges NH₃ einzubringen oder kaltes Melamin rückzuführen und dem abzukühlenden Melamin zuzumischen.

In einer Ausführungsform der Erfindung wird eine Melaminschmelze in einem Wirbelschichtapparat, der etwa bei 340 °C und 20 bar betrieben wird, mit kaltem, festem Melamin gekühlt. Das Wirbelbett wird mit NH₃ als Kreislaufgas betrieben. Eine NH₃-hältige Melaminschmelze (120 bar, 350 °C) wird in das Wirbelbett eingedüst und scheidet sich an den festen Melaminteilchen als Überzug aus verfestigtem Melamin ab. Die schwereren Teilchen werden im unteren Teil des Wirbelschichtapparates abgezogen und bei etwa 340 °C und 20 bar getempert. Anschließend wird über eine Druckschleuse auf Atmosphärendruck entspannt, in einem Kühlmischer rasch auf unter 150 °C gekühlt, in einer Mühle homogenisiert, überschüssiges NH₃ mit Luft ausgeblasen und in ein Silo gefördert.

Der besondere Vorteil des erfindungsgemäßen Verfahrens liegt demnach insbesondere darin, daß Melamin mit guter Qualität in einem einfacheren Verfahren erhalten wird, bei dem die Abkühlung des festen Melamins in üblichen Niederdruckapparaten erfolgt, die Verwendung von Hochdruckapparaten ist in diesem Verfahrensteil nicht mehr notwendig.

Mit Hilfe des erfindungsgemäßen Verfahrens kann Melamin mit einer Reinheit von über 99 Gew.%, bis zu 99,9 Gew.%, sowie entsprechend den Abkühlbedingungen (Kühlzeit, Entspannungsdruck, Entspannungstemperatur) mit geringem Melamgehalt (unter 300 ppm) sowie mit APHA-Werten als Maß für die Gelbstichkeit von unter 30, zumeist unter 20, bis zu 13 erhalten werden.

### Beispiel:

Ein aus einem Hochdruckprozeß stammendes Melaminpulver mit einem Melamgehalt von 2045 ppm wurde in einen Autoklaven eingebracht und während 60 min bei 340 °C und 20 bar NH₃-Druck verweilen gelassen. Anschließend wurde auf Atmosphärendruck entspannt und das Melamin während 10 sec, bzw. 1 min, bzw. 7,5 min bei 340 °C verweilen gelassen und anschließend durch Eintauchen des Autoklaven in eine Eis/Wasser-Mischung auf unter 150 °C gekühlt.

In einem Vergleichsbeispiel wurde Melaminpulver analog behandelt und entspannt, es wurde jedoch während 15 min bei 340 °C verweilen gelassen und anschließend durch Eintauchen des Autoklaven in eine Eis/Wasser-Mischung auf unter 150 °C gekühlt.

Die Reinheit des Melamins lag jeweils bei etwa 99,9 Gew.%. An den Proben wurden weiters der Melamgehalt und die Farbzahl (APHA) bestimmt. Der APHA-Wert wurde durch die Messung der Gelbfärbung des Melamin-Formaldehydharzes (molares Verhältnis Melamin : Formaldehyd = 1 : 3) auf einem Spektralphotometer (Hitachi U 2000) mit thermostatisierbarer Meßzelle, 5 cm Quarzglasküvette und verstellbarem Spektralbereich von 380 bis 640 nm als Funktion der Absorption bei 380 nm minus der Absorption bei 640 nm bestimmt.

| | Melam (ppm) | APHA |
|---|---|---|
| Verweilzeit bei 340 °C | | |
| 10 sec | 206 | 13 |
| 1 min | 223 | 13 |
| 7,5 min | 264 | 34 |
| | | |
| Vergleichsbeispiel: 15 min | 376 | 234 |

## Patentansprüche

1. Verfahren zur Herstellung von festem Melamin, **dadurch gekennzeichnet, daß**
a) eine NH₃-hältige Melaminschmelze durch Kühlen und/oder Entspannen, mit oder ohne NH3 Zufuhr verfestigt wird,
b) das erhaltene feste Melamin bei Temperaturen zwischen etwa 300 °C und seinem vom jeweiligen NH₃ Druck abhängigen Schmelzpunkt bei Drücken von etwa 10 bis 300 bar während 1 min bis 5 h verweilen gelassen wird,
c) anschließend auf einen Druck von etwa 1 bis 15 bar, bevorzugt auf etwa 1 bis 10 bar, entspannt wird,
d) rasch innerhalb von 0 bis 10 min auf eine Temperatur von unter 280 °C gekühlt wird und
e) anschließend in beliebiger Reihenfolge weiter gekühlt und weiter entspannt wird.

2. Verfahren gemäß Anspruch 1, **dadurch gekennzeichnet, daß** das feste Melamin gemäß c) auf einen Druck von etwa 1 bis 5 bar, bevorzugt auf etwa 1 bar (Atmosphärendruck) entspannt wird.

3. Verfahren gemäß Anspruch 1 oder 2, **dadurch gekennzeichnet, daß** gemäß d) rasch innerhalb von 10 sec bis 5 min, bevorzugt innerhalb von 1 bis 2 min auf unter 280 °C gekühlt wird.

4. Verfahren gemäß einem der Ansprüche 1 bis 3, **dadurch gekennzeichnet, daß** gemäß d) auf eine Temperatur von unter 250 °C, bevorzugt unter 200 °C, besonders bevorzugt unter 100 °C gekühlt wird.

5. Verfahren gemäß einem der Ansprüche 1 bis 4, **dadurch gekennzeichnet, daß**
a) eine NH₃-hältige Melaminschmelze in einem mit im wesentlichen aus NH₃ als Kreislaufgas betriebenen Wirbelbett durch Entspannung und Kühlung mit festem Melamin und/oder festen Inertstoffen verfestigt wird,
b) das erhaltene feste Melamin bei Temperaturen zwischen etwa 300 °C und seinem vom jeweiligen NH₃ Druck abhängigen Schmelzpunkt bei Drücken von etwa 10 bis 300 bar während 1 min bis 5 h verweilen gelassen wird,
c) anschließend auf einen Druck von etwa 1 bis 15 bar, bevorzugt auf etwa 1 bis 10 bar, entspannt wird,
d) rasch innerhalb von 0 bis 10 min auf eine Temperatur von unter 280 °C gekühlt wird und
e) anschließend in beliebiger Reihenfolge weiter gekühlt und weiter entspannt wird.

6. Verfahren gemäß einem der Ansprüche 1 bis 5, **dadurch gekennzeichnet, daß** die Kühlung des festen Melamins gemäß d) über Kühlflächen, Kühlschnecken oder Wärmetauscher erfolgt.

7. Verfahren gemäß einem der Ansprüche 1 bis 5, **dadurch gekennzeichnet, daß** die Kühlung des festen Melamins gemäß d) mit flüssigem NH₃ oder mit kalten Gasen, bevorzugt NH₃, Stickstoff oder Luft erfolgt.

8. Verfahren gemäß einem der Ansprüche 1 bis 5, **dadurch gekennzeichnet, daß** die Kühlung des festen Melamins gemäß d) in einem mit im wesentlichen aus NH₃ als Kreislaufgas betriebenen Wirbelbett mit kaltem, festem Melamin und/oder kalten festen Inertstoffen erfolgt.

## Claims

1. Process for preparing solid melamine, **characterized in that**
a) an NH₃-containing melamine melt is solidified by cooling and/or depressurization, with or without supply of NH₃,
b) the resultant solid melamine is allowed to remain for from 1 min to 5 h at pressures of from about 10 to 300 bar and at temperatures of from about 300°C to its melting point, which depends on the prevailing pressure of NH₃,
c) and this is followed by depressurization to a pressure of from about 1 to 15 bar, preferably from about 1 to 10 bar,
d) and rapid cooling takes place within a period of from 0 to 10 min to a temperature of less than 280°C, and
e) this is followed by further depressurization and further cooling in any desired sequence.

2. Process according to Claim 1, **characterized in that** the solid melamine is depressurized as in c) to a pressure of from about 1 to 5 bar, preferably about 1 bar (atmospheric pressure).

3. Process according to Claim 1 or 2, **characterized in that** rapid cooling takes place as in d) within a period of from 10 sec to 5 min, preferably within a period of from 1 to 2 min, to less than 280°C.

4. Process according to any of Claims 1 to 3, **characterized in that** cooling takes place as in d) to a temperature of less than 250°C, preferably less than 200°C, particularly preferably less than 100°C.

5. Process according to any of Claims 1 to 4, **characterized in that**
a) solid melamine and/or inert solid substances is/are used to solidify an NH₃-containing melamine melt in a fluidized bed operated using essentially NH₃ as circulating gas, by depressurization and cooling,
b) the resultant solid melamine is allowed to remain for from 1 min to 5 h at pressures of from about 10 to 300 bar and at temperatures of from about 300°C to its melting point, which depends on the prevailing pressure of NH₃,
c) and this is followed by depressurization to a pressure of from about 1 to 15 bar, preferably from about 1 to 10 bar,
d) and rapid cooling takes place within a period of from 0 to 10 min to a temperature of less than 280°C, and
e) this is followed by further depressurization and further cooling in any desired sequence.

6. Process according to any of Claims 1 to 5, **characterized in that** cooling surfaces, cooling screws, or heat exchangers are used to cool the solid melamine as in d).

7. Process according to any of Claims 1 to 5, **characterized in that** liquid NH₃ or low-temperature gases, preferably NH₃, nitrogen or air, is/are used to cool the solid melamine as in d).

8. Process according to any of Claims 1 to 5, **characterized in that** cold, solid melamine and/or cold inert solid substances is/are used to cool the solid melamine as in d) in a fluidized bed operated using essentially NH₃ as circulating gas.

## Revendications

1. Procédé pour la préparation de mélamine solide, **caractérisé en ce que**
a) une masse fondue de mélamine contenant du NH₃ est solidifiée par refroidissement et/ou détente, avec ou sans apport de NH₃,
b) la mélamine solide obtenue est abandonnée pendant 1 minute à 5 heures à des températures comprises entre environ 300°C et son point de fusion dépendant de la pression respective de NH₃, sous des pressions d'environ 10 à 300 bars,
c) ensuite est détendue jusqu'à une pression d'environ 1 à 15 bars, de préférence jusqu'à environ 1 à 10 bars,
d) est rapidement refroidie en l'espace de 0 à 10 minutes jusqu'à une température inférieure à 280°C et
e) ensuite, en un ordre quelconque, est refroidie davantage et détendue davantage.

2. Procédé selon la revendication 1, **caractérisé en ce que** la mélamine solide selon c) est détendue jusqu'à une pression d'environ 1 à 5 bars, de préférence jusqu'à environ 1 bar (pression atmosphérique).

3. Procédé selon la revendication 1 ou 2, **caractérisé en ce que** selon d) on refroidit rapidement jusqu'à une température inférieure à 280°C en l'espace de 10 secondes à 5 minutes, de préférence en l'espace de 1 à 2 minutes.

4. Procédé selon l'une quelconque des revendications 1 à 3, **caractérisé en ce que** selon d) on refroidit jusqu'à une température inférieure à 250°C, de préférence inférieure à 200°C, de façon particulièrement préférée inférieure à 100°C.

5. Procédé selon l'une quelconque des revendications 1 à 4, **caractérisé en ce que**
a) une masse fondue de mélamine contenant du NH₃ est solidifiée dans un lit fluidisé entraîné essentiellement par NH₃ en tant que gaz circulant, par détente et refroidissement avec de la mélamine solide et/ou des substances inertes solides,
b) la mélamine solide obtenue est abandonnée pendant 1 minute à 5 heures à des températures comprises entre environ 300°C et son point de fusion dépendant de la pression respective de NH₃, sous des pressions d'environ 10 à 300 bars,
c) ensuite est détendue jusqu'à une pression d'environ 1 à 15 bars, de préférence jusqu'à environ 1 à 10 bars,
d) est rapidement refroidie en l'espace de 0 à 10 minutes jusqu'à une température inférieure à 280°C et
e) ensuite, en un ordre quelconque, est refroidie davantage et détendue davantage.

6. Procédé selon l'une quelconque des revendications 1 à 5, **caractérisé en ce que** le refroidissement de la mélamine solide selon d) s'effectue au moyen de surfaces réfrigérantes, de serpentins refroidisseurs ou d'échangeurs thermiques.

7. Procédé selon l'une quelconque des revendications 1 à 5, **caractérisé en ce que** le refroidissement de la mélamine solide selon d) s'effectue avec du NH₃ liquide ou avec des gaz froids, de préférence NH₃, l'azote ou l'air.

8. Procédé selon l'une quelconque des revendications 1 à 5, **caractérisé en ce que** le refroidissement de la mélamine solide selon d) s'effectue dans un lit fluidisé entraîné essentiellement par NH₃ en tant que gaz circulant, avec de la mélamine solide froide et/ou des substances inertes solides froides.
